# EUROPEAN PATENT SPECIFICATION

(11) **EP 3 082 932 B1**
(45) Date of publication and mention of the grant of the patent: **31.01.2018**
(21) Application number: 14830437.1
(22) Date of filing: 18.12.2014
(51) Int. Cl.: A61M 25/01

(54) **STEERING TOOL**
LENKWERKZEUG
OUTIL DE PILOTAGE

(30) Priority: 19.12.2013 US 201361918073 P
(43) Date of publication of application: 26.10.2016
(73) Proprietor: Bendit Technologies Ltd., 4951024 Petah Tikva (IL)
(72) Inventor: CABIRI, Oz, 5029300 Ganot (IL)
(74) Representative: Becker Kurig Straus
(86) International application number: PCT/US2014/071075
(87) International publication number: WO 2015/095475

(56) References cited:
- US-A1- 2007 219 465
- US-A1- 2010 274 270
- US-A1- 2010 318 067
- US-A1- 2013 116 705

## Description

### FIELD OF THE INVENTION

The present invention generally relates to a steering tool for steering medical devices through body lumens. The closest prior art is document US 2013/0116705 A1, which defines the preamble of claim 1.

### BACKGROUND OF THE INVENTION

PCT Patent Application PCT/US2013/040691, to the present inventor, describes a steering tool for steering medical devices through body lumens. The steering tool has an internal tube disposed inside an external tube. The internal and external tubes are arranged for longitudinal axial movement relative to one another. The distal end of the internal tube is fixedly joined to the distal end of the external tube. One or both of the internal and external tubes is slotted near the distal end thereof. The longitudinal axial movement causes bending of the distal ends of the tubes. One or both of the internal and external tubes are slotted near the distal ends thereof. The steering tool provides a distal tip which combines steerability, flexibility and torqueability. The tool eliminates the need for pull/push wires.

Some of the advantages of that steering tool include reduced cross section, circular cross section in each direction for uniform stability of bending in different directions (towards two or more sides), very thin wall thickness, and applicability to very small tubes (e.g., diameters of 0.2-3 mm). The steering tool also works well with larger tubes. The steering tool simplifies production and reduces the number of parts for any steerable endoscope in medical and industrial fields.

### SUMMARY OF THE INVENTION

The present invention seeks to provide further improvements to the steering tool for steering medical devices through body lumens, as is described more in detail hereinbelow. The present invention is defined in claim 1 and further embodiments are defined in the dependent claims.

### BRIEF DESCRIPTION OF THE DRAWINGS

The present invention will be understood and appreciated more fully from the following detailed description taken in conjunction with the drawings in which:
Fig. 1 is a simplified illustration of a steering tool, showing one of the tubes of the steering tool in a spread-out view;
Fig. 2 is a simplified illustration of the steering tool, showing internal and external tubes which are slotted;
Fig. 3 is a simplified illustration of the steering tool, showing an alignment pin for proper alignment of the tubes during assembly;
Fig. 4 is a simplified illustration of the steering tool with the internal part being a hollow tube with no slots or a conduit for fluid or wires for energy delivery or optic fiber to transfer optic data or laser;
Figs. 5A and 5B are simplified pictorial and cutaway illustrations, respectively, of a steering tool with a manipulation handle, constructed and operative in accordance with a non-limiting embodiment of the present invention;
Figs. 5C, 5D and 5E are simplified pictorial, partially cutaway and sectional illustrations, respectively, of a steering tool with a manipulation handle, constructed and operative in accordance with another non-limiting embodiment of the present invention, Fig. 5E being taken along lines E-E in Fig. 5C;
Figs. 6A-6D are simplified illustrations of another way to join the internal and external tubes of the steering tool, wherein Fig. 6A shows the external tube in a flattened form, Figs. 6B and 6C show the external tube in its rolled, tubular form and Fig. 6D illustrates the internal tube joined to the external tube;
Figs. 7A, 7B and 7C are simplified illustrations of how the geometry of the tube slots can be used to achieve different effects and
Fig. 8 is a simplified illustration of shapes that can be achieved by varying the slots, such as a helical shape.

### DETAILED DESCRIPTION OF EMBODIMENTS

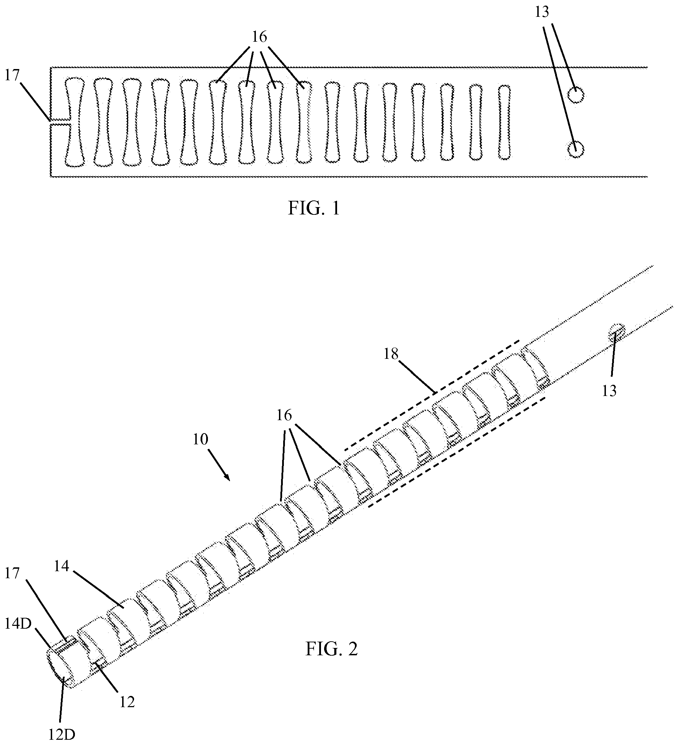

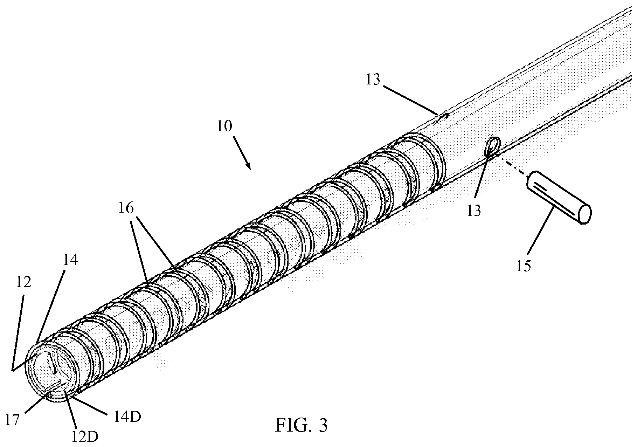

Reference is now made to Figs. 2 and 3, which illustrate a steering tool 10.

Steering tool 10 includes an internal tube 12 disposed inside an external tube 14. A distal end 12D of internal tube 12 is fixedly joined to a distal end 14D of external tube 14. The term "joined" encompasses any method for attaching the materials of the tubes together, such as but not limited to, welding, ultrasonic welding, thermal bonding, adhesive bonding, molding, and others. The internal and external tubes 12 and 14 are arranged for longitudinal axial movement relative to one another (except for their distal ends which are joined together). Internal tube 12 and external tube 14 are each formed with one or more alignment holes 13 for correct alignment (including axial and rotational alignment about the longitudinal axis) of the tubes during joining and assembly. An alignment pin 15 (Fig. 3) may be inserted in alignment holes 13 to hold the tubes in the proper alignment during joining. The alignment holes 13 may be off-center and/or of two different diameters to ensure that the tubes are not accidentally aligned incorrectly.

The outer diameter of internal tube 12 is smaller than the inner diameter of external tube 14 so they can easily slide relative to each other. This difference in diameter may pose a problem during welding or other types of joining. In order to achieve good joining (e.g., welding or bonding) between the tubes despite the difference in diameters, an open-ended axial slot 17 (also seen in the flattened view of Fig. 1) is formed in internal tube 12 and/or external tube 14. Slot 17 provides a path for welding, soldering or adhesive material to flow and join the tubes together.

Internal and external tubes 12 and 14 may be made of any suitably flexible, medically safe material, such as but not limited to, stainless steel (e.g., AISI 316), nitinol, cobalt-chromium alloy, nickel-titanium alloy, and others, glass fibers, plastics (e.g., nylon, polypropylene, and many others) or combinations thereof. As will be described further below, the tubes can be used for applications involving light guides, lasers, optic or electrical transfer and other uses, in addition to the mechanical function of bending.

At least one of the internal and external tubes 12 and 14 is slotted with slots 16 near the distal end thereof (e.g., transverse to the longitudinal axis of the tubes; the term transverse encompassing any angle - not just perpendicular - which is not parallel to the longitudinal axis of the tubes). In the preferred embodiment both tubes are slotted, but alternatively only one of the internal and external tubes is slotted and the other may be flexible but not slotted. The longitudinal axial movement causes bending of the distal ends of the tubes (on account of them being joined together), as is known from PCT/US2013/040691. One of the internal and external tubes can be longer than the other (e.g., the internal one is longer for grasping its proximal end for pushing and pulling thereof).

Slots 16 increase the flexibility toward the distal end of the tube or tubes for steerability of the device and controlled manipulation thereof. The amount of flexibility can be controlled by the number of slots, spacing therebetween, shape of the slot, angle subtended by the slot, thickness of the tube, material of the tube, and other factors. Slots 16 may subtend an arc of about 180-270°.

For example, as seen in Fig. 1, the shapes of slots 16 change as a parameter of distance from the distal end. In the illustrated example, the slots decrease in length and width (e.g., asymptotically to a minimum size) with increased distance from the distal end. In this manner, the bending radius remains basically constant at any distance from the distal end and the bending moment increases with increased distance from the distal end. Axial slot 17 may be open to the most distal slot 16.

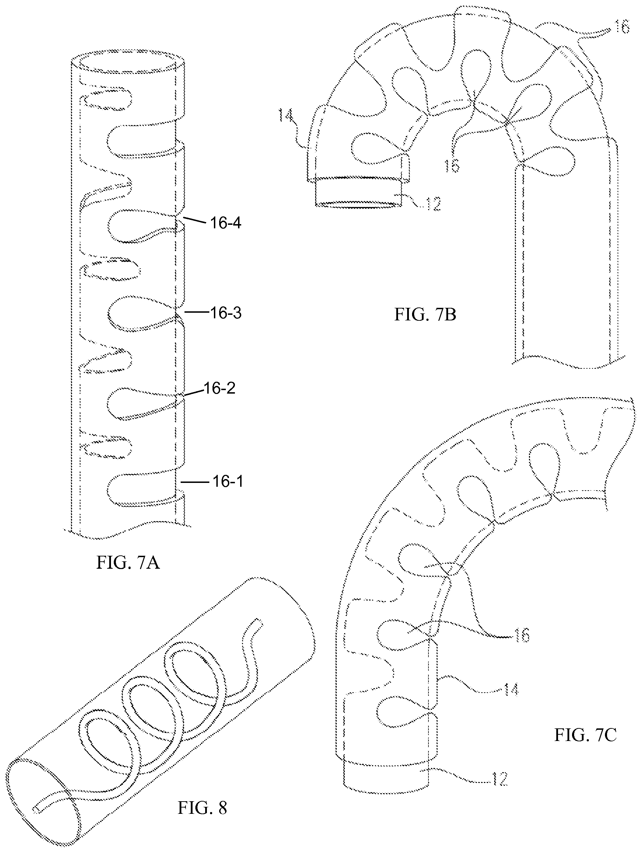

Another example of how the geometry of the slots can be used to achieve different effects is now explained with reference to Figs. 7A, 7B and 7C. Fig. 7A illustrates the steering tool in a straight position; Fig. 7B illustrates the steering tool bent to the left; and Fig. 7C illustrates the steering tool bent to the right. Both internal and external tubes 12 and 14 can be slotted with slots 16, wherein the inner tube slots have open ends facing in one direction, while the outer tube slots have the open ends facing in another direction 180° from each other. In addition, the slots of one of the tubes are spaced apart more than the slot spacing of the other tube. Alternatively, the inner or outer tube can be made such that some of the slots 16 have open ends facing in one direction, while other slots 16 have the open ends facing in another direction, such as 180° apart. In addition, the slots of one side of the tube are spaced apart more than the slot spacing of the other side. Fig. 7A also shows another option, in which some of the slots are shaped differently than others. For example, the open ends of slots 16-1, 16-2, 16-3 and 16-4 are differently shaped - some are more closed than others. This means some of the slots will stop bending before others stop bending, because the open ends of the slot abut against each other in the bent position, thus serving as a hard stop to prevent further bending. By using differently shaped slots, different bending effects can be achieved.

The tubes can bend towards the open side of the slots until eventually the slots become closed or "pinched", at which point the closed slot serves as a stopper that permits no further bending. Due to the fact that one set of the slots is spaced further from each other than the slot spacing in the other set of slots, the tube can bend more in one direction than the other and can bend differently when the tube is pushed as opposed to pulled. The same effect can be achieved by different slot radii or shapes. Accordingly, in the embodiments of Figs. 7A-7C, some of slots 16 of internal and/or external tubes 12 and 14 are more bendable than other slots 16 of internal and/or external tubes 12 and 14 which are less bendable.

Variation of the angle between the two sets of slots (that is, not 180°) enables achieving different bending shapes, forces and sensitivity for different sides of the tubes. Fig. 8 illustrates another example of shapes that can be achieved by varying the slots, namely, a helical shape, which may be useful for self-centering in a blood vessel or other lumen.

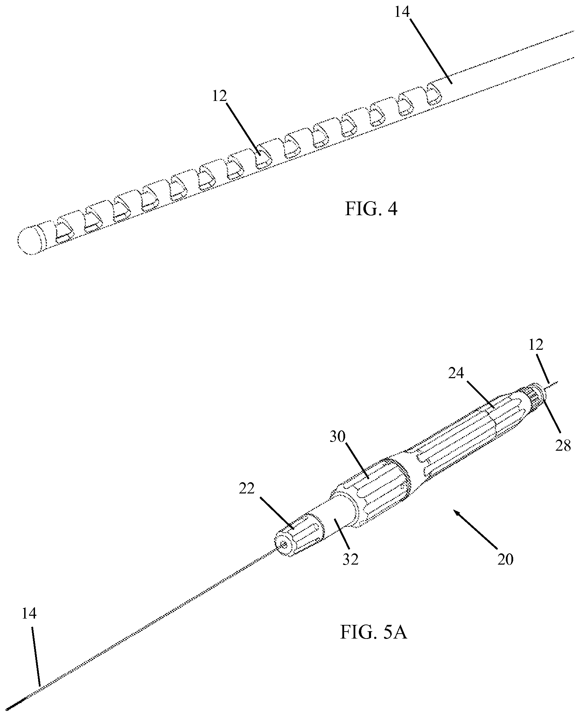

The steering tool 10 may be covered with a semi-rigid or flexible sheath 18 (shown partially in broken lines in Fig. 2) and used as a catheter or needle. In a more preferred embodiment, shown in Fig. 4, the internal tube 12 is a simple tube or wire with no slots. In the case of a hollow tube, the internal tube 12 can serve as the conduit for delivery of fluids, light, laser, optic, cameras, illumination, electrical energy transfer (e.g., wired connections), and others, in addition to the mechanical function of bending, Thus, the device can be used to deliver fluids to places in a body with high accuracy, such as but not limited to, direct injection of drugs into the brain. For example, tool 10 may be used as a needle to protrude through or into a blood vessel and inject substances directly into the brain, tumor or infected area. The steering tool 10 may be used as a catheter to direct and deliver cooled gas to freeze tumors or other areas. The steering tool 10 may be used as a catheter to guide fiber optic or laser devices for illumination, treatment, ablation or drying or other uses. In another embodiment, the fiber optic can be part of the mechanical steering system, serving as a pull wire inside the internal or external tube. In this manner, a controlled fiber optic with a diameter of 0.3 mm becomes feasible. The distal edge shape of the internal tube, external tube and/or overall tool may be not only circular, but also shaped as an electrode, needle or other shapes.

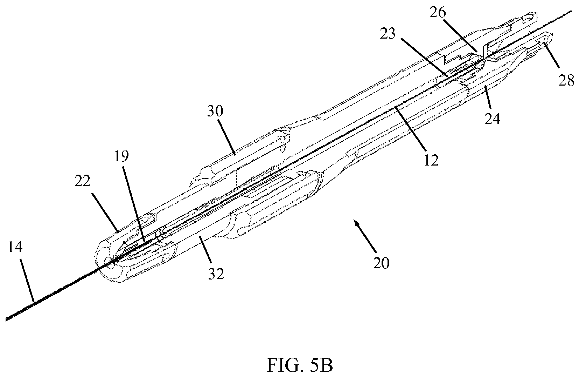

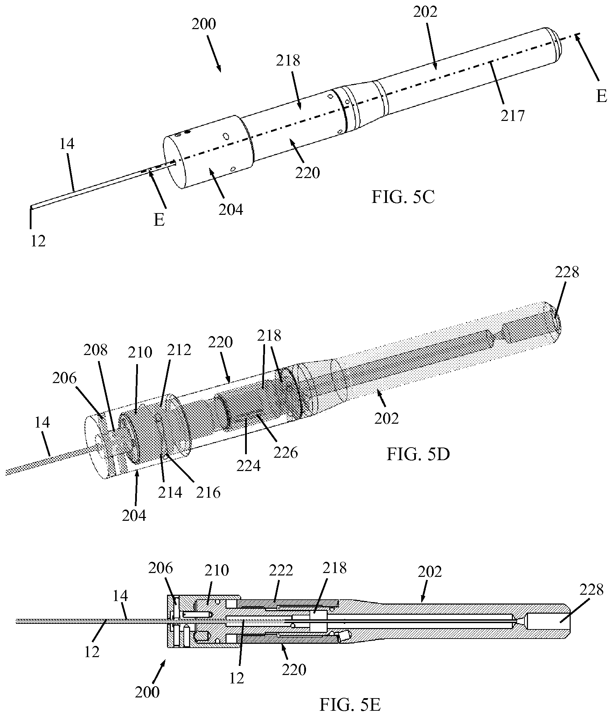

Reference is now made to Figs. 5A and 5B, which illustrate a steering tool with a manipulation handle 20, constructed and operative in accordance with a non-limiting embodiment of the present invention.

In this steering tool, the proximal end 19 of the external tube 14 is affixed or locked in an external tube holder 22 at the distal end of the handle 20. The internal tube 12 is disposed in external tube 14 as described above. The internal tube 12 extends through the length of handle 20, and a proximal portion 23 of internal tube 12 may be held by a proximal internal tube holder 24. The internal tube holder 24 may include a septum 26 which may be used to seal passage of the internal tube 12 therethrough. The handle 20 may be provided with a proximal fluid connector 28.

The handle 20 is provided with a tube manipulator 30, such as a linear slider, for causing relative axial movement of the internal and external tubes. For example, the tube manipulator 30 may abut against or be connected to a movable portion 32 of handle 20, which is connected to or abuts against the external tube holder 22. By moving tube manipulator 30 distally, the movable portion 32 also moves distally and causes the internal tube 12 to move distally as well, thereby causing bending of the distal tip of the internal and external tubes, as described above. The tube manipulator 30 may abut proximally against a stationary portion of the handle 20 and/or may "click" into a groove formed in the handle, which serves as a proximal stop and locks the tube manipulator 30 in place. The tube manipulator 30 may be released when desired from the locked position and relocked in place. There is free movement during insertion (handle unlocked) of the steering tool into the body lumen, so that both tubes can move freely and allow the steering tool to bend in accordance with the shape of the body lumen (such as any kind of curved path). After reaching the desired destination in the body lumen, the handle is locked for no more bending. Thus, the steering tool can be used very easily as a guide wire for guiding catheters and other devices.

Reference is now made to Figs. 5C-5E, which illustrate a steering tool 200 with a manipulation handle 202, constructed and operative in accordance with another non-limiting embodiment of the present invention. This embodiment may be used with any of the internal and external tubes described in the other embodiments.

In this embodiment, as in the embodiment of Figs. 5A-5B, the internal tube 12 and the external tube 14 are held in a manipulation handle that can axially move one of the tubes with respect to the other tube along their longitudinal axes to cause bending of the common distal tips of the tubes. The embodiment of Figs. 5C-5E differs from the previous embodiment, in that the internal tube 12 and the external tube 14 are held in a manipulation handle that additionally rotates one of the tubes with respect to the other tube around their longitudinal axes. This twists one of the tubes with respect to the other tube and causes the tubes to take on a bent or twisted shape not achievable with the previous handle. With the combination of staggered or phase-shifted slots, the tubes can take on an S-bend shape, helical shape and many other curved and twisted shapes. (It is possible to generate the phase shift by rotating the tubes and/or by cutting the slots with phase shift; in any case the bending shape occurs with the axial movement.) The user can twist the tubes as desired and the tool allows locking or otherwise maintaining the tubes at the selected twisted orientation. The description that follows is for the illustrated embodiment, but the principles of this embodiment can be carried out with other configurations and structures.

As seen in Fig. 5D, in steering tool 200, the proximal end of the external tube 14 is affixed or locked in an external tube holder 204 at the distal end of the handle 202. The external tube holder 204 has fasteners 206 (e.g., pins, screws or the like) that pass through a distal hub 208 and clamp on the outside contour of external tube 14. Hub 208 axially extends from a drum 210 housed inside external tube holder 204. The external tube holder 204, together with hub 208 and drum 210, rotates around the longitudinal axis 217 of the tool. The proximal face of drum 210 interfaces with a disc 212 by means of catches, such as detents 214 that selectively sit in grooves 216. As external tube holder 204 is rotated around the longitudinal axis of the tool, the detents get caught in the grooves ("click" into the grooves). In this manner, the user can twist the tubes as desired and the detents lock or otherwise maintain the tubes at the selected twisted orientation.

The internal tube 12 is disposed in external tube 14 as described above. The internal tube 12 extends through the external tube holder 204 and is fixed or held by a proximal internal tube holder 218. Disc 212 is fixedly connected to or part of internal tube holder 218. The handle 202 is provided with a tube manipulator 220 for causing relative axial movement of the internal and external tubes. For example, the tube manipulator 220 may be a sleeve 222 attached to internal tube holder 218. Sleeve 222 may be threadedly connected with external tube holder 204. As sleeve 222 is rotated, the internal tube 12 advances or retracts axially with respect to external tube 14, thereby causing bending of the distal tip of the internal and external tubes, as described above. A guide pin 224 may be provided that slides in a guide slot 226, for guiding axial movement of the tube. The proximal end of the tool may have an inlet opening 228 for passing guidewires, optical fibers or other tools through the tubes.

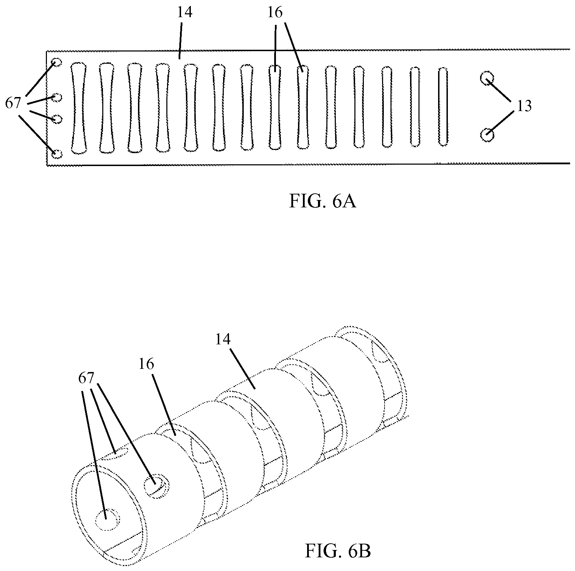

As mentioned above with reference to Figs. 1-3, in order to achieve good joining (e.g., welding or bonding) between the tubes despite the difference in diameters, open-ended axial slot 17 is formed in internal tube 12 and/or external tube 14. Reference is now made to Figs. 6A-6D, which illustrate another way to join internal and external tubes 12 and 14.

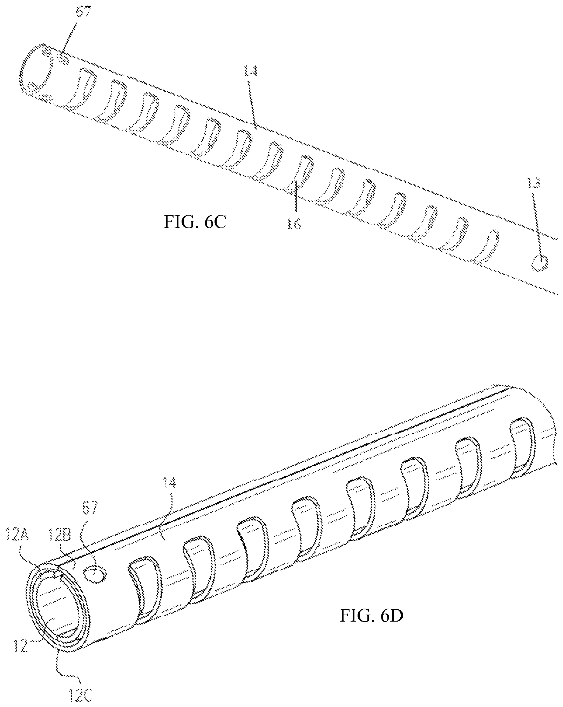

In this example, external tube 14 is formed with apertures 67, which may be round holes (as in the illustrated embodiment) or may be openings of any size and shape, such as elongate slots, oval cutouts, diamond-shaped cutouts and more. Fig. 6A shows external tube 14 in a flattened form. Figs. 6B and 6C both show external tube 14 in its rolled, tubular form.

Fig. 6D illustrates internal tube 12 joined to external tube 14. The welding (e.g., laser welding in metal tubes) or bonding (e.g., adhesive or thermal in plastic tubes) of the internal and external tubes 12 and 14 is done at or through apertures 67. It is noted that internal tube 12 may be rolled from a flat sheet so that it has edges 12A and 12B which are close to or abut each other. Apertures 67 may be advantageously located near edges 12A and 12B, that is, on both sides of the cut internal tube 12. Joining the tubes at this position of the apertures helps prevent the edges 12A and 12B from springing back away from each other. The stiffer side of the internal tube 12 opposite the junction of the edges 12A and 12B (designated 12C in Fig. 6D) may be joined to the external tube 14 with a single aperture 67 instead of two apertures as in the illustrated example. Apertures 67 of external tube 14 are located near the stiffest and the most flexible areas of the internal tube (12A, 12B and 12C) in order to transfer shear forces but not in between them were torque exists.

In yet another example, the assembly of the inner and outer tubes can operate without connecting/welding or bonding the ends of the tubes to one another. Instead, one of the tubes can be made with a distal stopper at its distal end. The second tube can slide relative to the first tube until the second tube abuts the distal stopper of the first tube and then further application of force causes the tubes to bend as described above. Such a construction is very efficient for one-sided bending. Unlike the examples which have the tubes affixed to each other at the distal portions, this example will work only when pushing one of the tubes towards the distal end but will not work for pulling the tube proximally.

## Claims

1. A steering tool (10, 200) comprising:
an internal tube (12) disposed inside an external tube (14), said internal and external tubes (12, 14) being arranged for longitudinal axial movement relative to one another, wherein a distal end of said internal tube (12) is fixedly joined to a distal end of said external tube (14), and at least one of said internal and external tubes (12, 14) is formed with transverse slots (16) near the distal end thereof, and wherein the longitudinal axial movement causes bending of the distal ends of said tubes;
wherein said internal and external tubes (12, 14) are held in internal and external tube holders (218, 204; 22, 24), respectively, which are mounted in a manipulation handle (20, 202), and wherein said internal and external tube holders (218, 204; 22, 24) are movable axially with respect to one another along their longitudinal axes to cause bending of the distal ends of said tubes, and wherein said internal and external tube holders (218, 204; 22, 24) are rotatable with respect to one another around their longitudinal axes; wherein said handle (20) is provided with a tube manipulator (30) for causing relative axial movement of said internal and external tubes (12, 14), wherein said tube manipulator (30) abuts against or is connected to a movable portion (32) of said handle (20), which is connected to or abuts against said external tube holder (22), and wherein said tube manipulator (30) is arranged to abut proximally against a stationary portion of said handle (20), **characterised in that** said tube manipulator (30) is configured to selectively interact with a groove formed in said handle(2), wherein said groove is adapted to serve as a proximal stop in said handle (20) and lock said tube manipulator (30) in place.

2. The steering tool (10, 200) according to claim 1, wherein said external tube holder (204) interfaces with said internal tube holder (218) with catches (214, 216) that maintain said internal and external tubes (12, 14) at a selected twisted orientation.

3. The steering tool (10, 200) according to claim 1, wherein a proximal end of the tool has an inlet opening (228) for passing therethrough tools.

4. The steering tool (10, 200) according to claim 1, wherein some of said slots (16) of said at least one of said internal and external tubes (12, 14) are more bendable than other slots (16) of said at least one of said internal and external tubes (12, 14) which are less bendable.

5. The steering tool (10, 200) according to claim 1, wherein said slots (16) which are less bendable abut against a stop (16) that prevents from bending more than the slots (16)

6. The steering tool (10, 200) according to claim 1, wherein said at least one of said internal and external tubes (12, 14) bends differently when pushed as opposed to pulled.

7. The steering tool (10, 200) according to claim 1, wherein some of said slots (16) of said at least one of said internal and external tubes (12, 14) have open ends facing in one direction, while other slots (16) of said at least one of said internal and external tubes (12, 14) have open ends facing in another direction, the slots (16) of one side of said at least one of said internal and external tubes (12, 14) are spaced apart more than slots (16) of another side of said at least one of said internal and external tubes(12, 14).

8. The steering tool (10, 200) according to claim 1, wherein said external tube (14) is formed with apertures (67) for joining to said internal tube (12).

9. The steering tool (10, 200) according to claim 8, wherein said apertures (67) are located near the stiffest and the most flexible areas of said internal tube (12A, 12B and 12C).

10. The steering tool (10, 200) according to claim 1, wherein some of said slots (16) of said at least one of said internal and external tubes (12, 14) have open ends facing in one direction, while other slots (16) of said at least one of said internal and external tubes (12, 14) have open ends facing in another direction, the slots (16) of one side of said at least one of said internal and external tubes (12, 14) are spaced apart more than slots (16) of another side of said at least one of said internal and external tubes(12, 14).

11. The steering tool (10, 200) according to claim 10, wherein said external tube (14) is formed with apertures (67) for joining to said internal tube (12).

12. The steering tool (10, 200) according to claim 11, wherein said apertures (67) are located near the stiffest and the most flexible areas of said internal tube (12A, 12B and 12C).

13. The steering tool (10, 200) according to claim 1, wherein said internal tube holder (24) comprises a septum (26) to seal passage of said internal tube (12) therethrough.

14. The steering tool (10, 200) according to claim 1, wherein said handle (20) comprises a proximal fluid connector (28).

## Patentansprüche

1. Lenkwerkzeug (10, 200), umfassend:
ein inneres Rohr (12), das innerhalb eines äußeren Rohres (14) angeordnet ist, wobei das innere und das äußere Rohr (12, 14) für eine axiale Längsbewegung relativ zueinander ausgelegt sind, wobei ein distales Ende des inneres Rohres (12) fest mit einem distalen Ende des äußeren Rohres (14) verbunden ist und wenigstens eines von dem inneren und dem äußeren Rohr (12, 14) mit Querschlitzen (16) in der Nähe seines distalen Endes ausgebildet ist, und wobei die axiale Längsbewegung ein Biegen der distalen Ende der Rohre verursacht;
wobei das innere und das äußere Rohr (12, 14) in einem inneren bzw. einem äußeren Rohrhalter (218, 204; 22, 24) gehalten werden, welche in einem Handhabungsgriff (20, 202) angebracht sind, und wobei der innere und der äußere Rohrhalter (218, 204; 22, 24) relativ zueinander entlang ihrer Längsachsen axial beweglich sind, um ein Biegen der distalen Enden der Rohre zu verursachen, und wobei der innere und der äußere Rohrhalter (218, 204; 22, 24) relativ zueinander um ihre Längsachsen drehbar sind, wobei der Griff (20) mit einem Rohrmanipulator (30) zum Verursachen einer relativen axialen Bewegung des inneren und des äußeren Rohres (12, 14) versehen ist, wobei der Rohrmanipulator (30) an einen beweglichen Abschnitt (32) des Griffs (20) anstößt oder mit einem solchen verbunden ist, welcher mit dem äußeren Rohrhalter (22) verbunden ist oder an diesen anstößt, und wobei der Rohrmanipulator (30) dafür ausgelegt ist, proximal an einen stationären Abschnitt des Griffs (20) anzustoßen, **dadurch gekennzeichnet, dass** der Rohrmanipulator (30) dafür eingerichtet ist, selektiv mit einer Rille zu interagieren, die in dem Griff (2) ausgebildet ist, wobei die Rille dafür ausgelegt ist, als ein proximaler Anschlag in dem Griff (20) zu dienen und den Rohrmanipulator (30) in seiner Position zu verriegeln.

2. Lenkwerkzeug (10, 200) nach Anspruch 1, wobei der äußere Rohrhalter (204) mit dem inneren Rohrhalter (218) mit Mitnehmern (214, 216) verbunden ist, welche das innere und das äußere Rohr (12, 14) in einer gewählten Drehausrichtung halten.

3. Lenkwerkzeug (10, 200) nach Anspruch 1, wobei ein proximales Ende des Werkzeugs eine Einlassöffnung (228) zum Hindurchführen von Werkzeugen durch sie aufweist.

4. Lenkwerkzeug (10, 200) nach Anspruch 1, wobei einige der Schlitze (16) des wenigstens einen von dem inneren und dem äußeren Rohr (12, 14) stärker biegsam als andere Schlitze (16) des wenigstens einen von dem inneren und dem äußeren Rohr (12, 14) sind, welche weniger biegsam sind.

5. Lenkwerkzeug (10, 200) nach Anspruch 1, wobei die Schlitze (16), welche weniger biegsam sind, an einen Anschlag (16) anstoßen, wodurch sie stärker am Biegen gehindert werden als die Schlitze (16), welche stärker biegsam sind.

6. Lenkwerkzeug (10, 200) nach Anspruch 1, wobei sich das wenigstens eine von dem inneren und dem äußeren Rohr (12, 14), wenn es geschoben wird, anders biegt als dann, wenn es gezogen wird.

7. Lenkwerkzeug (10, 200) nach Anspruch 1, wobei einige der Schlitze (16) des wenigstens einen von dem inneren und dem äußeren Rohr (12, 14) offene Enden aufweisen, die in eine Richtung gewandt sind, während andere Schlitze (16) des wenigstens einen von dem inneren und dem äußeren Rohr (12, 14) offene Enden aufweisen, die in eine andere Richtung gewandt sind, wobei die Schlitze (16) einer Seite des wenigstens einen von dem inneren und dem äußeren Rohr (12, 14) mehr voneinander beabstandet sind als Schlitze (16) einer anderen Seite des wenigstens einen von dem inneren und dem äußeren Rohr (12, 14).

8. Lenkwerkzeug (10, 200) nach Anspruch 1, wobei das äußere Rohr (14) mit Öffnungen (67) zum Verbinden mit dem inneren Rohr (12) ausgebildet ist.

9. Lenkwerkzeug (10, 200) nach Anspruch 8, wobei sich die Öffnungen (67) in der Nähe des steifsten und des flexibelsten Bereichs des inneren Rohres (12A, 12B und 12C) befinden.

10. Lenkwerkzeug (10, 200) nach Anspruch 1, wobei einige der Schlitze (16) des wenigstens einen von dem inneren und dem äußeren Rohr (12, 14) offene Enden aufweisen, die in eine Richtung gewandt sind, während andere Schlitze (16) des wenigstens einen von dem inneren und dem äußeren Rohr (12, 14) offene Enden aufweisen, die in eine andere Richtung gewandt sind, wobei die Schlitze (16) einer Seite des wenigstens einen von dem inneren und dem äußeren Rohr (12, 14) mehr voneinander beabstandet sind als Schlitze (16) einer anderen Seite des wenigstens einen von dem inneren und dem äußeren Rohr (12, 14).

11. Lenkwerkzeug (10, 200) nach Anspruch 10, wobei das äußere Rohr (14) mit Öffnungen (67) zum Verbinden mit dem inneren Rohr (12) ausgebildet ist.

12. Lenkwerkzeug (10, 200) nach Anspruch 11, wobei sich die Öffnungen (67) in der Nähe des steifsten und des flexibelsten Bereichs des inneren Rohres (12A, 12B und 12C) befinden.

13. Lenkwerkzeug (10, 200) nach Anspruch 1, wobei der innere Rohrhalter (24) eine Trennwand (26) umfasst, um den Durchgang des inneren Rohres (12) durch sie abzudichten.

14. Lenkwerkzeug (10, 200) nach Anspruch 1, wobei der Griff (20) einen proximalen Fluidanschluss (28) umfasst.

## Revendications

1. Outil de pilotage (10,200) comprenant :
un tube interne (12) disposé à l'intérieur d'un tube externe (14), lesdits tubes interne et externe (12, 14) étant disposés de manière à exécuter un mouvement axial longitudinal l'un par rapport à l'autre, une extrémité distale dudit tube interne (12) étant assemblée fixement à une extrémité distale dudit tube externe (14), et au moins un desdits tubes interne et externe (12, 14) étant conformé avec des fentes transversales (16) près de son extrémité distale, et le mouvement axial longitudinal provoquant la pliure des extrémités distales desdits tubes ;
lesdits tubes interne et externe (12, 14) étant respectivement maintenus dans des supports de tubes interne et externe (218, 204; 22, 24) qui sont montés dans une poignée de manipulation (20, 202), et lesdits supports de tubes interne et externe (218, 204; 22, 24) étant mobiles axialement l'un par rapport à l'autre le long de leurs axes longitudinaux pour provoquer la pliure des extrémités distales desdits tubes, et lesdits supports de tubes interne et externe (218, 204; 22,24) pouvant tourner l'un par rapport à l'autre autour de leurs axes longitudinaux ; ladite poignée (20) étant pourvue d'un manipulateur de tube (30) pour provoquer un mouvement axial relatif desdits tubes interne et externe (12, 14), ledit manipulateur de tubes (30) butant contre ou étant connecté à une partie mobile (32) de ladite poignée (20) qui est connectée au ou bute contre ledit support de tube et externe (22), et ledit manipulateur de tube (30) étant conçu pour buter au niveau proximal contre une section stationnaire de ladite poignée (20), **caractérisé en ce que** ledit manipulateur de tube (30) est conçu pour interagir sélectivement avec une gorge pratiquée dans ladite poignée (2), ladite gorge étant apte à servir d'arrêt proximal dans ladite poignée (20) et à bloquer ledit manipulateur de tube (30) en place.

2. Outil de pilotage (10, 200) selon la revendication 1, dans lequel ledit support de tube externe (204) est en interface avec ledit support de tube interne (218) par des crochets (214, 216) qui maintiennent lesdits tubes interne et externe (12, 14) dans une orientation en torsion sélectionnée.

3. Outil de pilotage (10, 200) selon la revendication 1, dans lequel une extrémité proximale de l'outil comporte une ouverture d'entrée (228) pour y faire passer des outils.

4. Outil de pilotage (10, 200) selon la revendication 1, dans lequel certaines desdites fentes (16) desdits au moins un tubes interne et externe (12, 14) sont plus pliables que d'autres fentes (16) desdits au moins un tubes interne et externe (12, 14) qui sont moins pliables.

5. Outil de pilotage (10, 200) selon la revendication 1, dans lequel lesdites fentes (16) qui sont moins pliables butent contre un arrêt (16) qui empêche de plier davantage les fentes (16).

6. Outil de pilotage (10, 200) selon la revendication I, dans lequel lesdits au moins un tubes interne et externe (12, 14) se plient différemment quand ils sont poussés par opposition à quand ils sont tirés.

7. Outil de pilotage (10, 200) selon la revendication 1, dans lequel certaines desdites fentes (16) desdits au moins un tubes interne et externe (12, 14) ont des extrémités ouvertes tournées dans un sens, alors que d'autres fentes (16) desdits au moins un tubes interne et externe (12, 14) ont des extrémités ouvertes tournées dans un autre sens, les fentes (16) d'une face desdits au moins un tubes interne et externe (12, 14) étant plus espacées que les fentes (16) d'une autre face desdits au moins un tubes interne et externe (12, 14).

8. Outil de pilotage (10, 200) selon la revendication I, dans lequel ledit tube externe (14) est réalisé avec des ouvertures (67) pour l'assembler audit tube interne (12).

9. Outil de pilotage (10, 200) selon la revendication 8, dans lequel lesdites ouvertures (67) sont situées près des zones les plus rigides et les plus flexibles dudit tube interne (12A, 12B et 12C).

10. Outil de pilotage (10, 200) selon la revendication 1, dans lequel certaines desdites fentes (16) desdits au moins un tubes interne et externe (12, 14) ont des extrémités ouvertes tournées dans un sens, alors que d'autres fentes (16) desdits au moins un tubes interne et externe (12, 14) ont des extrémités ouvertes tournées dans un autre sens, les fentes (16) d'une face desdits au moins un tubes interne et externe (12, 14) étant plus espacées que les fentes (16) d'une autre face desdits au moins un tubes interne et externe (12, 14).

11. Outil de pilotage (10, 200) selon la revendication 10, dans lequel ledit tube externe (14) est réalisé avec des ouvertures (67) pour l'assembler audit tube interne (12).

12. Outil de pilotage (10, 200) selon la revendication 11, dans lequel lesdites ouvertures (67) sont situées près des zones les plus rigides et les plus flexibles dudit tube interne (12A, 12B et 12C).

13. Outil de pilotage (10, 200) selon la revendication 1, dans lequel ledit support de tube interne (24) comprend un septum (26) pour colmater le passage dudit tube interne (12) à travers celui-ci.

14. Outil de pilotage (10, 200) selon la revendication 1, dans lequel ladite poignée (20) comprend un connecteur proximal de fluide (28).
